# NEUE EUROPÄISCHE PATENTSCHRIFT

(11) **EP 2 688 522 B2**
(45) Veröffentlichungstag und Bekanntmachung des Hinweises auf die Entscheidung über den Einspruch: **16.01.2019**
(45) Hinweis auf die Patenterteilung: 16.12.2015
(21) Anmeldenummer: 12711781.0
(22) Anmeldetag: 23.03.2012
(51) Int. Cl.: A61F 2/50, A61F 2/74, A61F 2/66

(54) **PROTHESENFUSSEINSATZ UND PROTHESENFUSS**
PROSTHETIC FOOT INSERT AND PROSTHETIC FOOT
INSERT DE PIED ARTIFICIEL ET PIED ARTIFICIEL

(30) Priorität: 23.03.2011 DE 102011014994
(43) Veröffentlichungstag der Anmeldung: 29.01.2014
(73) Patentinhaber: Otto Bock Healthcare GmbH, 37115 Duderstadt (DE)
(72) Erfinder: MOSLER, Lüder, 37115 Duderstadt (DE); MÖNICKE, Carsten, 04838 Laussig (DE)
(74) Vertreter: Stornebel, Kai
(86) Internationale Anmeldenummer: PCT/EP2012/001282
(87) Internationale Veröffentlichungsnummer: WO 2012/126633

(56) Entgegenhaltungen:
- EP-A1- 2 420 212
- US-A- 5 116 384
- US-A1- 2006 015 192
- US-A1- 2006 069 450
- US-A1- 2010 042 228
- US-A1- 2010 312 360

## Beschreibung

Die Erfindung betrifft einen Prothesenfußeinsatz mit oberen Anschlussmitteln, einer sich von den oberen Anschlussmitteln nach vorn erstreckenden Dachfeder sowie einer Basisfeder, die eine im Vorderfußbereich zum Boden konvexe Kontur aufweist und an zumindest zwei Stellen mit der Dachfeder gekoppelt ist, wobei ein hinteres Koppelelement zur Abstützung der Basisfeder vorgesehen und zwischen dem Koppelelement, der Dachfeder und der Basisfeder ein Freiraum ausgebildet ist. Die Erfindung betrifft ebenfalls einen Prothesenfuß mit einem Prothesenfußeinsatz und einer Prothesenfußkosmetik.

Die EP 1 322 265 B1 beschreibt einen Fußeinsatz für einen Kunstfuß mit einer oberen, etwa dachförmig ausgebildeten Feder, die im Dachspitzenbereich einen Adapteranschluss und hiervon ausgehend eine konkav nach unten durchgebogene, sich bis in den Fersenbereich erstreckenden Fersenfeder sowie eine konkav nach unten durchgebogene, sich bis in den Vorderfußbereich erstreckende Vorfußfeder aufweist. Eine Basisfeder ist an den freien Schenkelenden der Fersenfeder und der Vorfußfeder verbunden, so dass ein Einfederraum ausgebildet wird. Die Basisfeder und die Vorfußfeder sind hinsichtlich der Form- und Biegelastizität so ausgelegt, dass sich unter Einwirkung einer zunehmenden Belastung im Vorderfußbereich in diesem Bereich die Vorfußfeder und die Basisfeder sukzessive aneinander anlegen. Ein solcher Prothesenfußeinsatz weist gute Gebrauchseigenschaften auf, stellt aber hohe Anforderungen an den zu verwendenden Werkstoff.

Die EP 1 357 867 B1 beschreibt eine Unterschenkelprothese mit einer oberen Fußplatte und einer unterhalb davon angeordneten Vorfußplatte sowie einer Fersenplatte, die mit der oberen Fußplatte über eine elastomere Zwischenschicht verbunden sind, wobei die elastomere Zwischenschicht die Vorfußplatte und die Fersenplatte voneinander beabstandet hält. Der Spalt zwischen der Vorfußplatte und der Fersenplatte befindet sich in einem mittleren Fußabschnitt und gewährleistet, dass sich die Platten unabhängig voneinander bewegen können.

Die US 2006/0069450 A1 betrifft einen Prothesenfußeinsatz mit einem unteren Fußteil mit kurvenartiger Formgebung und einem oberen Fußteil, das kürzer als das untere Fußteil ausgebildet ist. Das obere Fußteil und das untere Fußteil sind aus einem faserverstärkten Kunststoff hergestellt und über eine Zwischenschicht, die als federndes Element ausgebildet ist, miteinander verbunden.

Die US 2010/0312360 A1 betrifft einen Prothesenfußeinsatz mit oberen Anschlussmitteln, die an einer Vorderfußfeder befestigt sind. Die Vorderfußfeder ist mit einer Fersenfeder gekoppelt. Eine Basisfeder ist mit dem vorderen Ende der Vorfußfeder und dem unteren Ende der Fersenfeder gekoppelt, wobei zwischen der Befestigungsstelle der Fersenfeder und der Vorfußfeder an der Basisfeder ein Freiraum ausgebildet ist.

Weiterhin ist von der Firma Otto Bock ein Prothesenfußeinsatz mit einer durchgehenden oberen Feder und einer geteilten unteren Feder bekannt. Der vordere Bereich der unteren Feder und der hintere Bereich der unteren Feder sind voneinander getrennt und über ein durchgehendes Elastomerelement an der oberen Feder befestigt. Die Fersennachgiebigkeit wird durch die Elastomeranbindung hergestellt, die Vorderfußnachgiebigkeit aus dem Zusammenwirken der oberen Feder und der unteren Federn sowie dem Elastomerelement.

Ein weiteres Modell eines Prothesenfußeinsatzes der Firma Otto Bock sieht eine durchgehende obere Feder und eine durchgehende untere Feder vor, die eine durchgehende Elastomeranbindung aufweisen. Die Fersennachgiebigkeit wird durch den hinteren Teil der unteren Feder und das Elastomerelement bereitgestellt, die Nachgiebigkeit im Vorderfußbereich aus der oberen und der unteren Feder sowie dem Elastomerelement. Ein solcher Prothesenfußeinsatz besitzt eine geringe Aufbauhöhe, dadurch bedingt aber auch einen steifen Vorderfuß. Die Fersenfunktion ist relativ weich und weist, bedingt durch eine geringe freie Federlänge, eine erhebliche Progression auf.

Passive Prothesenfüße machen zwischen Steh- und Geheigenschaften einen Kompromiss, da ihnen die Möglichkeit eines muskulären Ausgleichs fehlt. Für eine verbesserte Stehsicherheit werden deshalb Füße mit einem rigiden Vorfußhebel hergestellt, während Füße für bessere Geheigenschaften weichere Vorfußhebel aufweisen. Aktive Prothesenfüße können aufgrund von Messwerten entscheiden, welche Aktivität vorliegt und dementsprechend die Eigenschaften verändern und sich auf die jeweilige Aktivität einstellen.

Aufgabe der vorliegenden Erfindung ist es, einen Prothesenfußeinsatz und einen Prothesenfuß bereitzustellen, der gute Steh- und Geheigenschaften aufweist und auch mit weniger belastbaren Werkstoffen eine gute Belastbarkeit erzielt.

Erfindungsgemäß wird diese Aufgabe durch einen Prothesenfußeinsatz mit den Merkmalen des Hauptanspruches gelöst. Vorteilhafte Ausgestaltungen und Weiterbildungen der Erfindung sind in den Unteransprüchen, den Figuren und der Beschreibung offenbart.

Der erfindungsgemäße Prothesenfußeinsatz mit oberen Anschlussmitteln, einer sich von den oberen Anschlussmitteln nach vorn erstreckenden Dachfeder sowie einer vorzugsweise zugstarren Basisfeder, die an zumindest zwei Stellen mit der Dachfeder gekoppelt ist, wobei ein hinteres Koppelelement zur Abstützung der Basisfeder vorgesehen und zwischen dem Koppelelement, der Dachfeder und der Basisfeder ein Freiraum ausgebildet ist, sieht vor, dass im Fersenbereich die Basisfeder nach hinten über das hintere Koppelelement als freier Hebel hinausragt. Durch das Überstehen der Basisfeder, die vorzugsweise zugstarr und als eine durchgängige Blattfeder ausgebildet ist, nach hinten über das hintere Koppelelement hinaus entsteht ein freier Hebel, der bei einer Fersenlast eine 3-Punkt-Biegung der Basisfeder entstehen lässt und dadurch deren elastischen Eigenschaften besser ausnutzt. Das hintere Koppelelement, das die Abstützung der Basisfeder an der Dachfeder oder den oberen Anschlussmitteln bewirkt, dient als Gelenkpunkt oder Gelenkeinrichtung, um die die Basisfeder herum schwingen kann. Dabei ist aufgrund der in der Regel durchgängigen Ausgestaltung des hinteren Koppelelementes als eine Strebe oder dergleichen eine elastische Biegung innerhalb des Koppelelementes gegeben. Für eine erleichterte Biegung ist eine dünne Ausgestaltung des Koppelelementes oder eine Art Filmscharnier durch eine gezielt eingebrachte Schwächung in dem Koppelelement vorteilhaft.

Die Basisfeder kann eine im Vorderfußbereich zum Boden konvexe, also nach unten gekrümmte Kontur aufweisen, um das Abrollverhalten zu beeinflussen. Alternativ kann eine Prothesenkosmetik verwendet werden, die eine eingearbeitete Kontur aufweist und die zusammen mit der Basisfeder die konvexe Kontur bereitstellt.

Eine Weiterbildung der Erfindung sieht vor, dass sich an den nach unten gewölbten Vorderfußbereich der Basisfeder ein nach oben gewölbter Mittelfußbereich anschließt, so dass eine wellenartige untere Kontur der Basisfeder erreicht wird. Von vorne nach hinten gesehen verläuft die Kontur in der Seitenansicht zunächst nach unten abfallend bis zu einem Tiefpunkt oder einem tiefen Bereich im Vorderfußbereich, danach erfolgt ein Anstieg bis in den Mittelfußbereich und danach wieder ein Abfallen bis in den Fersenbereich, wobei das Ende der Basisfeder im Fersenbereich ein nach oben ansteigendes Endstück aufweisen kann.

Das hintere Koppelelement kann als eine gewölbte Fersenfeder oder als ein Elastomerelement ausgebildet sein.

Die Dachfeder kann kürzer als die Basisfeder ausgebildet sein, wodurch sich ein besonders kompakter Aufbau des Prothesenfußeinsatzes realisieren lässt.

Eine Weiterbildung, die nicht Gegenstand der Erfindung ist, sieht vor, dass ein vorderes Koppelelement an einem vorderen Bereich der Dachfeder angeordnet ist und die Basisfeder das vordere Koppelelement nach vorne überragt. Dadurch ist es möglich, dass bei einer Vorfußbelastung die Federkraft und Durchbiegung der Basisfeder nicht nur durch den vor dem vorderen Koppelelement angeordneten Teil der Basisfeder vorgenommen werden muss, sondern dass eine Durchbiegung des hinter dem vorderen Koppelelement liegenden Teils der Basisfeder von der Dachfeder weg erfolgt, so dass die gesamte Basisfeder an der Federung teilnimmt. Aufgrund der Biegung der Basisfeder im Mittelfußbereich von der Dachfeder weg erfolgt eine weitere Biegung oder Verschwenkung um das hintere Koppelelement bzw. um eine Schwenkachse, die von dem hinteren Koppelelement ausgebildet wird. Dadurch ist es möglich, auch das hintere Koppelelement an der Aufnahme von Biegekräften bei einer Vorfußbelastung zu beteiligen.

Das hintere Koppelelement bildet eine Schwenkachse für den freien Hebel aus, die im Wesentlichen senkrecht zu der Gehrichtung und parallel zu der Basisfeder verläuft. Dadurch wird bei einem Fersenauftritt zunächst der freie, nach hinten über das Koppelelement hinausstehende Hebel um die Schwenkachse innerhalb des hinteren Koppelelementes verformt. Aufgrund der Befestigung der Basisfeder an der Dachfeder und dem dadurch zu dem hinteren Koppelelement beabstandeten zweiten Koppelpunkt der Basisfeder an der Dachfeder kommt es zu einer Durchbiegung des mittleren Abschnitts der Basisfeder von der Dachfeder weg, so dass auch der zwischen den Koppelelementen angeordnete Teil der Basisfeder an der Federungsarbeit bei einem Fersenauftritt teilnimmt. Dadurch können weniger belastbare Materialien eingesetzt werden, da sich die Belastung auf eine größere Federlänge verteilt.

Die Basisfeder kann um eine Torsionsachse parallel zu der Längserstreckung der Basisfeder verschwenkbar an der Dachfeder oder an den oberen Anschlussmitteln gelagert sein. Diese Torsionsachse kann durch die Koppelelemente oder ein Koppelelement bereitgestellt werden, so dass eine Verlagerbarkeit der Basisfeder relativ zu der Dachfeder um die Torsionsachse möglich ist. Dadurch können Unebenheiten des Bodens bei einem Auftritt ausgeglichen werden, ebenfalls kann eine gewünschte Nachgiebigkeit realisiert werden, die sich bei einem leicht schrägen Auftreten während des Gehens als vorteilhaft herausgestellt hat.

Das hintere Koppelelement kann mit dem hinteren Ende der Dachfeder abschließen, zumindest steht es nicht nach hinten über die Dachfeder über, so dass ein kompakter Aufbau des Prothesenfußeinsatzes durch eine relativ kurze Dachfeder und im Wesentlichen bündig mit der Dachfeder abschließenden Koppelelementen vorgesehen sind. Der freie Hebel der Basisfeder ergibt sich dann aus dem Überstand der Basisfeder nach hinten über das hintere Koppelelement.

Das vordere Koppelelement ist als Elastomerelement ausgebildet und zwischen dem vorderen Endbereich der Basisfeder und dem nach oben ansteigenden Teil der Basisfeder angeordnet.

Der Aufbau der Basisfeder und der Dachfeder mit den Koppelelementen ist so gewählt, dass bei einer Vorderfußbelastung der von diesen Komponenten gebildete Freiraum verkleinert wird, indem sich die Dachfeder und die Basisfeder aufeinander zu bewegen.

Eine Weiterbildung der Erfindung sieht vor, dass zwischen der Basisfeder und der Dachfeder eine Vakuumpumpe angeordnet ist. Die Anordnung der Vakuumpumpe zwischen der Basisfeder und der Dachfeder ermöglicht es, die Vakuumpumpe von strukturellen Lasten zu befreien, da diese von den übrigen Komponenten des Prothesenfußeinsatzes übernommen werden. Die Relativbewegung zwischen der Basisfeder und der Dachfeder ermöglicht es, einen Pumpenhub zu realisieren, der ausreichend ist, um ein Vakuum zu erzeugen, beispielsweise um Luft aus einem Prothesenschaft abzusaugen und so den Prothesenschaft sicher an einem Liner festzulegen.

Die Vakuumpumpe kann als Koppelelement ausgebildet oder ein Teil eines Koppelelementes sein. Als Koppelelement kommt insbesondere das hintere Koppelelement in Frage, wenn es als ein Elastomerelement oder ein Federelement ausgebildet ist, da bei einem Fersenstoß relative hohe Verformungsenergien aufgenommen werden, die zu einem entsprechend hohen Pumpenhub führen können. Das Koppelelement selber bildet mit seiner elastischen Rückstellkraft die notwendige Gegenkraft, um eine Rückstellbewegung in die Ausgangsposition zu erreichen. Durch eine entsprechende Ausgestaltung des Koppelelementes ist es möglich, auch eine Hubbegrenzung in beiderlei Richtung herzustellen, also sowohl bei der Kompression als auch bei der Elongation des Koppelelementes. Das Koppelelement übernimmt somit auch Zugkräfte und dient damit als Hubbegrenzung im Rahmen der Rückstellbewegung. Ebenfalls ist es vorgesehen und möglich, dass das vordere Koppelement Teil der Vakuumpumpe ist oder als solche ausgebildet ist, auch wenn während eines Gangzyklus' im Vorderfußbereich eine geringere Impulshöhe als im Fersenbereich zu erwarten ist.

Die Erfindung betrifft ebenfalls einen Prothesenfuß mit einem Prothesenfußeinsatz, wie er vorstehend beschrieben worden ist, mit einer Prothesenfußkosmetik, die einen Sohlenbereich mit unterschiedlichen Materialstärken aufweist. Wenn der Sohlenbereich der Fußprothesenkosmetik im Vorderfußbereich und/oder Fersenbereich dicker als im Mittelfußbereich ausgebildet ist, ergibt sich ein zusätzlicher Federungseffekt und eine vorteilhafte Abrollkontur.

Nachfolgend werden Ausführungsbeispiele der Erfindung anhand der beigefügten Figuren näher erläutert. Es zeigen:
Figur 1 - eine schematische Seitenansicht eines Prothesenfußeinsatzes, der nicht Gegenstand der Erfindung ist;
Figur 2 - eine erfindungsgemäße Variante der Figur 1;
Figur 3 - eine weitere Ausgestaltung eines Prothesenfußeinsatzes, die nicht Gegenstand der Erfindung ist;
Figur 4 - eine Seitenansicht eines Prothesenfußes;
Figur 5 - eine Variante der Figur 4, die nicht Gegenstand der Erfindung ist; sowie
Figur 6 - einen Prothesenfußeinsatz mit einer Vakuumpumpe.

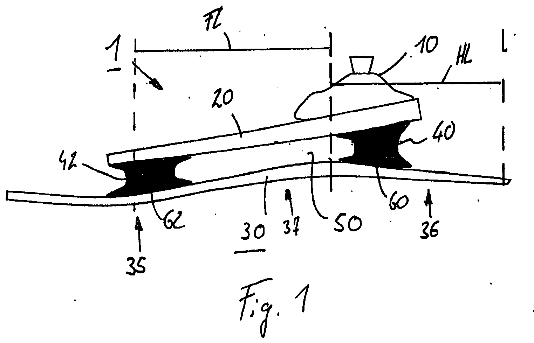

In Figur 1 ist in einer schematischen Seitenansicht ein Prothesenfußeinsatz 1 mit oberen Anschlussmitteln 10 in Gestalt eines Adapterpylons dargestellt. Über die oberen Anschlussmittel 10 ist es möglich, den Prothesenfußeinsatz 1 an ein Unterschenkelrohr einer Protheseneinrichtung anzuschließen. Der Prothesenfußeinsatz 1 weist eine Dachfeder 20 auf, die sich von dem oberen Anschlussmittel 10 schräg nach vorne erstreckt. Das obere Anschlussmittel 10 ist an der Dachfeder 20 befestigt. An dem hinteren Ende der Dachfeder 20 ist ein Koppelelement 40 angeordnet, das eine bikonkave Form aufweist, also zwei nach innen gewölbte Krümmungen. An der Dachfeder 20 und dem hinteren Koppelelement 40 ist eine Basisfeder 30 angeordnet, die zugstarr ausgebildet ist und als eine Blattfederkonstruktion in der dargestellten Ausführungsform vorliegt. Die Basisfeder 30 ist durchgängig und erstreckt sich ununterbrochen über die gesamte Länge, so dass sich ein geschlossener Freiraum 50 zwischen der Dachfeder 20, dem hinteren Koppelelement 40 und der Basisfeder 30 ausbildet.

Die Basisfeder 30 weist im dargestellten Ausführungsbeispiel einem Vorderfußbereich 35 auf, an dem sich ein Mittelfußbereich 37 anschließt, der ungefähr unterhalb der oberen Anschlussmittel 10 endet. An den Mittelfußbereich 37 schließt sich ein Fersenbereich 36 an. Die untere Kontur der Basisfeder 30 ist wellenförmig ausgebildet und sieht von dem vorderen Ende ausgehend zunächst eine zum Boden konvexe Krümmung vor, also eine Krümmung, die eine Wölbung nach unten aufweist. Die Wölbung kann entweder stetig oder auch abschnittsweise ausgebildet sein, wobei eine durchgängig gekrümmte Oberfläche oder Kontur den Vorteil eines glatten Abrollens aufweist. Eine Abflachung, beispielsweise im Bereich des Vorderfußes 35, stellt eine Verbesserung der Standsicherheit dar. Nach der Kontaktstelle oder Kontaktlinie der Basisfeder 30 mit dem Boden setzt sich die Krümmung weiterhin fort, so dass sich die Kontur der Unterseite der Basisfeder 30 von dem Boden entfernt und ein Freiraum ausgebildet wird, der ungefähr im Bereich des Fußgewölbes eines natürlichen Fußes angesiedelt ist und diesem entspricht. Im Mittelfußbereich 37 ist eine konkave, in Richtung zur Dachfeder 20 gewölbte Kontur ausgebildet, die in den Fersenbereich 36 übergeht. Der Fersenbereich 36 kann abgeflacht oder eine konvexe Krümmung aufweisen, um den Fersenstoß beim Aufsetzen des Prothesenfußeinsatzes durch Abrollen leicht aufnehmen zu können. Das hintere Ende der Basisfeder 30 kann eine konvexe Krümmung aufweisen. Der Krümmungsverlauf von vom nach hinten sieht also zunächst eine konvexe und dann konkave und dann wiederum konvexe Formgebung vor.

Die Dachfeder 20 endet ungefähr in der Mitte des Vorderfußbereiches 35 und bildet eine angenähert gerade Verbindung des oberen Federbogens 20 zu dem oberen Anschlussmittel 10. Die Dachfeder 20 wirkt somit als eine Strebe, die direkt zum oberen Anschlussmittel 10 des Prothesenfußeinsatzes 1 führt, so dass die während des Stehens und Gehens auf den Vorderfußbereich 35 wirkenden Kräfte auf die oberen Anschlussmittel 10 übergeleitet werden. Der Figur 1 ist weiterhin zu entnehmen, dass die Basisfeder 30 im Fersenbereich 36 über die Zusammenführungsstelle mit dem hinteren Koppelelement 40 hinaussteht. Das hintere Koppelelement 40 dient zur Abstützung der Basisfeder 30 und des sogenannten Fersenhebels HL, der sich von der Position des natürlichen Knöchelgelenks nach hinten erstreckt, der Vorderfußhebel FL erstreckt sich von der fiktiven Senkrechten durch die Position des Knöchelgelenks nach vorne. Durch das Überstehen des Fersenbereiches 36 über die Anbindungsstelle des hinteren Koppelelementes 40 an der Basisfeder 30 ist es möglich, dass bei einer Fersenlast eine 3-Punkt-Biegung der Basisfeder 30 entsteht, so dass die elastischen Eigenschaften der Basisfeder 30 besser ausgenutzt werden. Das hintere Koppelelement 40 dient somit als Drehpunkt oder Drehachse 60 für die Basisfeder 30 und bewirkt eine elastische Rückstellkraft, wenn eine Biegung der Basisfeder an dem hinteren Koppelelement 40 bei einem Fersenstoß bewirkt wird. Das hintere Koppelelement 40 wirkt somit als ein Gelenkpunkt oder eine Gelenkstelle für die Basisfeder 30 und trägt zum Federungsverhalten bei, wodurch es möglich ist, dass auch Materialien mit einer relativ geringen spezifischen Belastbarkeit für die Federn oder Koppelelemente eingesetzt werden können, so dass der Prothesenfußeinsatz insgesamt preiswerter produziert werden kann.

In der Ausführungsform gemäß der Figur 1 ist gezeigt, dass die Dachfeder 20 und die Basisfeder 30 als separate Blattfedern ausgebildet sind. Das obere Anschlussmittel 10 ist an der oberen Dachfeder 20 befestigt. Die Dachfeder 20 und die Basisfeder 30 sind über zwei separate Elastomerelemente 40, 42, beispielsweise aus einem Polyurethan, aneinander gekoppelt. Das vordere Elastomerelement 42 und das hintere Elastomerelement 40 dient jeweils als ein Koppelelement 40, 42, so dass zwischen den beiden Koppelelementen 40, 42 und den beiden Federn 20, 30 ein Freiraum 50 ausgebildet ist, der ungefähr im Mittelfußbereich 37 der Basisfeder 30 angeordnet ist.

Die Basisfeder 30 ist mit einer unteren Kontur ausgebildet, die eine konvexe Form im Vorderfußbereich 35, eine konkave Form im Mittelfußbereich 37 und eine im vorliegenden Ausführungsbeispiel geradlinige Form im Fersenbereich 36 aufweist. Statt der dargestellten geradlinigen Ausgestaltung der Basisfeder 30 im Fersenbereich 36 kann diese auch eine konvexe Form aufweisen.

Das vordere Koppelelement 42 ist ungefähr im Ballenbereich an der Basisfeder 30 festgelegt, das vordere Ende der im Vergleich zu der Basisfeder 30 kürzeren Dachfeder 20 endet ebenfalls ungefähr auf Höhe des Ballenbereichs 35 der Basisfeder 30, also in dem Bereich, der bei einem unbelasteten Prothesenfußeinsatz 1 auf dem Boden aufliegt und den Vorderfußbereich 35 abstützt. Durch die Lagerung der Basisfeder 30 an zwei separaten Koppelelementen 40, 42, die als Elastomerelemente ausgebildet sind, ist es möglich, sowohl bei einer Fersenlast als auch bei einer Vorderfußlast eine 3-Punkt-Biegung der Basisfeder 30 entstehen zu lassen, die darüber hinaus über die elastischen Koppelelemente 40, 42 eine zusätzliche Abfederung und Dämpfung erhält. Durch die besondere Ausgestaltung mit einem überstehenden Federarm im Fersenbereich 36 ist es möglich, die elastischen Eigenschaften der Basisfeder 30 besser auszunutzen.

Die Anordnung des vorderen Koppelelementes 42 im Bereich des Ballens in der konvexen Krümmung der Basisfeder 30 bewirkt eine hohe Strukturfestigkeit und Stabilität des Prothesenfußeinsatzes 1, wenn nach dem Fersenstoß der Vorderfuß den Boden berührt, da zusammen mit dem Aufsetzen des Ballenbereiches 35 die Dachfeder 20 wirksam wird. Durch den relativ unmittelbaren Einsatz der Dachfeder 20 nach dem vollständigen Aufsetzen des Prothesenfußes erhöht sich schlagartig die wirksame Gesamtfedersteifigkeit und damit die Kontrollierbarkeit des Prothesenfußes während des Kontaktes des Ballenbereiches 35 mit dem Boden. Im Verlauf der weiteren Abrollbewegung wird der Zehenbereich der Basisfeder 30 stärker belastet, insbesondere gegen Ende der Abrollphase, vor dem so genannten "toe off", wobei die Hauptfederwirkung durch die Basisfeder 30 aufgebracht wird. Da die Dachfeder 20 nur einen geringen Federanteil beiträgt, ist ein relativ weiches Abrollen möglich. Je nach Positionierung des vorderen Koppelelementes kann somit die Abrollcharakteristik verändert werden, eine Anordnung im Ballenbereich 35 hat einen stabilen Auftritt und eine weiche Abrollcharakteristik zur Folge, eine Anordnung weiter in Richtung des vorderen Endes der Basisfeder 30 ein weiches Auftreten und eine härtere Abstoßcharakteristik.

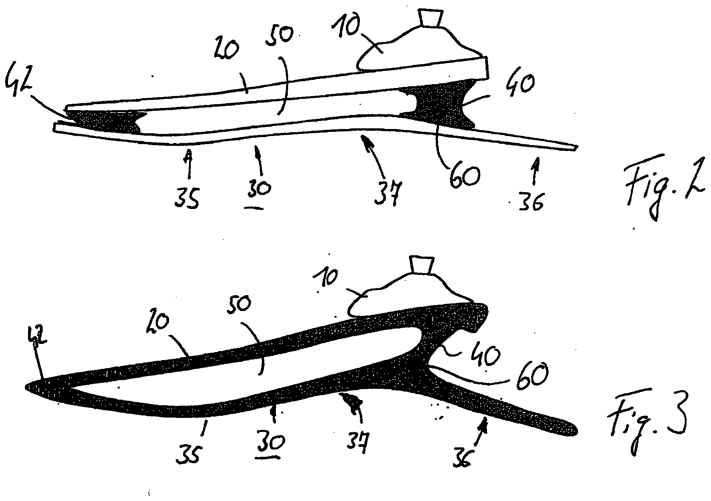

Eine Variante der Figur 1 ist in der Figur 2 dargestellt, die einen im Wesentlichen gleichen Aufbau aufweist, jedoch erstreckt sich die Dachfeder 20 bis zum vorderen Ende der Basisfeder 30. Die Kopplung der Dachfeder 20 an die Basisfeder 30 erfolgt über das vordere Koppelelement 42 im Zehenbereich der Basisfeder, was bei einer Vorfußlast zu einer Ballenauflage als dritten Lagerpunkt für eine 3-Punkt-Biegung der Basisfeder 20 bewirkt. Ein solcher Prothesenfußeinsatz vermittelt eine bessere Kontrolle in der Endphase der Standphase. Die Ausgestaltung der Koppelelemente 40, 42 als Elastomerelemente ermöglicht eine Verschwenkung oder Verkippung um eine Achse parallel zu der Längsachse des Prothesenfußeinsatzes. Ebenfalls ist eine Biegung um eine Schwenk- oder Drehachse 60 im Bereich des hinteren Koppelelementes möglich. In der Figur 1 ist ebenfalls eine zweite Schwenkachse 62 in dem vorderen Koppelelement 42 eingezeichnet, so dass bei einer Fersenbelastung an dem Kontaktpunkt mit dem Boden und den beiden Achsen 60, 62 eine Biegung der gesamten Basisfeder 30 erfolgen kann, so dass die komplette Basisfeder 30 zur Abfederung genutzt werden kann. Gleiches gilt auch für eine Vorderfußbelastung Bei der Variante gemäß Figur 2 liegt die vordere Schwenkachse in dem vorderen Koppelelement 42.

In der Figur 3 ist eine weitere Variante der Prothesenfußeinsatzes, die nicht Gegenstand der Erfindung ist, dargestellt. Hierbei ist eine einstückige Ausgestaltung der Dachfeder 20 und der Basisfeder 30 zusammen mit dem hinteren Koppelelement 40 gewählt. Auch hier ragt die Basisfeder 30 im Fersenbereich 36 über die Ankoppelstelle des hinteren Koppelelementes 40 nach hinten hinaus. Die Ankoppelung der Dachfeder 20 an die Basisfeder 30 erfolgt im Zehenbereich durch ein Verschmelzen, so dass der Zusammenführungsbereich der beiden Federn als Koppelelement 42 dient. Auch hier sind die Dachfeder 20, das hintere Koppelelement 40 sowie die Basisfeder 30 so ausgebildet, dass ein Freiraum 50 umschlossen wird, der seitlich zugänglich sein kann. Die untere Kontur der Basisfeder 30 sieht wie bei den Figuren 2 und 3 eine konvexe Vorfußform und eine konkave Form im Mittelfußbereich 37 vor, der Fersenbereich 36 der Basisfeder 30 kann geradlinig oder konvex ausgeformt sein. In dem dargestellten Ausführungsbeispiel ist der Prothesenfußeinsatz 1 bis auf die oberen Anschlussmittel 10 einstückig ausgebildet, insbesondere in einem Spritzgießverfahren mit einem geeigneten Polymerwerkstoff hergestellt. Die hintere Schwenkachse 60 liegt dabei in dem als Steg ausgebildeten, hinteren Koppelelement 40, die vordere Schwenkachse ist nicht dargestellt und liegt in der Verbindungsstelle der Dachfeder 20 mit der Basisfeder 30. Die Gelenkfunktion des hinteren Koppelelementes 40 kann durch eine gezielte Formgebung, beispielsweise eine Materialschwächung in dem Bereich, in dem die Drehachse liegen soll, unterstützt werden, so dass die Basisfeder 30 um das hintere Koppelelement 40 bei der Biegung verschwenken kann. Eine entsprechende Ausgestaltung des vorderen Koppelelementes 42 erleichtert das Verschwenken bei einer Vorderfußbelastung.

Die Anordnung des vorderen Koppelelementes 42 vor dem Ballenbereich 35 wie in der Figur 2 oder in der vorderen Spitze des Prothesenfußeinsatzes, wie in der Figur 3, ermöglicht ein weiches Auftreten, da sich die Basisfeder 30 mit der zum Boden konvexen Krümmung nach oben hin verlagern und verbiegen kann, so dass im Wesentlichen die Basisfeder 30 allein die Kräfte aufnimmt und weiterleitet. Erst bei einer Zehenbelastung, in der Regel kurz vor dem sogenannten "toe off", wird die Dachfeder 20 spürbar aktiviert, was zu einer Erhöhung des Gesamtfederwiderstandes am Ende der Abrollphase führt. Es kann sich mit dieser Variante kraftvoller abgedrückt werden, als wenn die Dachfeder 20 nicht mit einbezogen wäre.

Durch die Anbindung der Basisfeder 30 über das hintere Koppelelement 40 in Gestalt des Steges und das vordere Koppelelement 42 in Gestalt der Verbindung der Basisfeder 30 mit der Dachfeder 20 kann durch den nach hinten überstehenden Fersenbereich 36 der Basisfeder 30 zusätzlich zu der innerhalb des Fersenbereich 36 auftretenden Biegung eine Durchbiegung der Basisfeder 30 realisiert werden, wodurch die Federeigenschaften der Materialien besser ausgenutzt werden können.

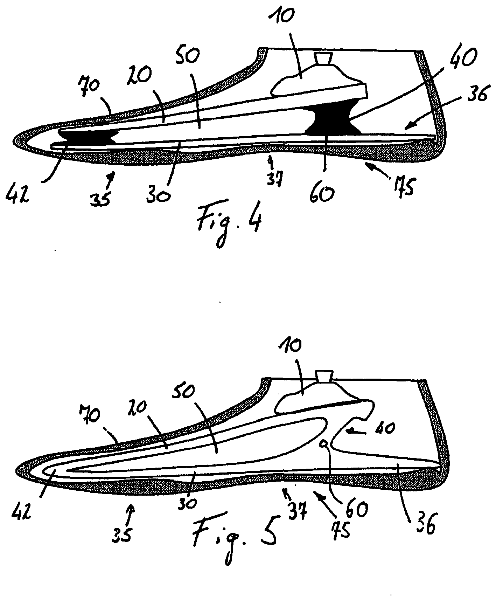

In der Figur 4 ist in einer Seitenansicht eine Variante eines Prothesenfußes mit einem Prothesenfußeinsatz gemäß Figur 2 dargestellt. Statt einer Basisfeder 30 mit einer geschwungenen Kontur, wie sie in der Figur 2 dargestellt ist, ist in der Figur 4 die Variante mit einer im Wesentlichen geraden Basisfeder 30 dargestellt. Die Basisfeder 30 ist auch hier über elastische Koppelelemente 40, 42 mit der Dachfeder 20 verbunden und schließt einen Freiraum 50 zwischen sich ein. Statt der konvexen Kontur der Basisfeder 30 im Vorderfußbereich 35 ist eine konturierte Prothesenkosmetik 70 vorgesehen, die im Vorderfußbereich 35 eine Verdickung aufweist, die zusammen mit der Basisfeder 30 eine konturierte, zum Boden gerichtete konvexe Abrollkontur bereitstellt. Die Prothesenkosmetik 70, die aus einem elastischen Werkstoff, beispielsweise Kunststoff, Silikon oder Gummi, besteht, weist auch im Fersenbereich 36 eine Verdickung auf, so dass zusammen mit der im Fersenbereich 36 ebenfalls geraden und im Wesentlichen mit einer konstanten Dicke ausgebildeten Basisfeder 30 auch hier eine konvexe, zum Boden gerichtet gewölbte Gesamtkontur aus dem Sohlenbereich 75 der Prothesenkosmetik 70 und der Basisfeder 30 verwirklicht wird.

Entsprechend der Ausgestaltung in der Figur 4 ist in der Figur 5 eine weitere Variante des Prothesenfußes, die nicht Gegenstand der Erfindung ist, gezeigt, bei der die Basisfeder 30 und die Dachfeder 20 einstückig ausgebildet sind. Auch in der Figur 5 ist die Basisfeder 30 im Wesentlichen gradlinig ausgebildet, so dass sich eine ebene Unterseite ausbildet. Die konvexe Kontur im Vorderfußbereich 35 und im Fersenbereich 36 wird durch das Zusammenwirken der gradlinigen Basisfeder 30 mit den verdickten Bereichen in dem Sohlenbereich 75 der Prothesenkosmetik 70 realisiert, wobei im Vorderfußbereich 35 und im Fersenbereich 36 eine im Verhältnis zum Mittelfußbereich 37 größere Materialstärke des Sohlenbereiches 75 vorhanden ist. Die vergrößerte Materialstärke kann auch durch separate Elemente, die an der Basisfeder 30 oder der Prothesenfußkosmetik 70 angeordnet sind, bereitgestellt werden.

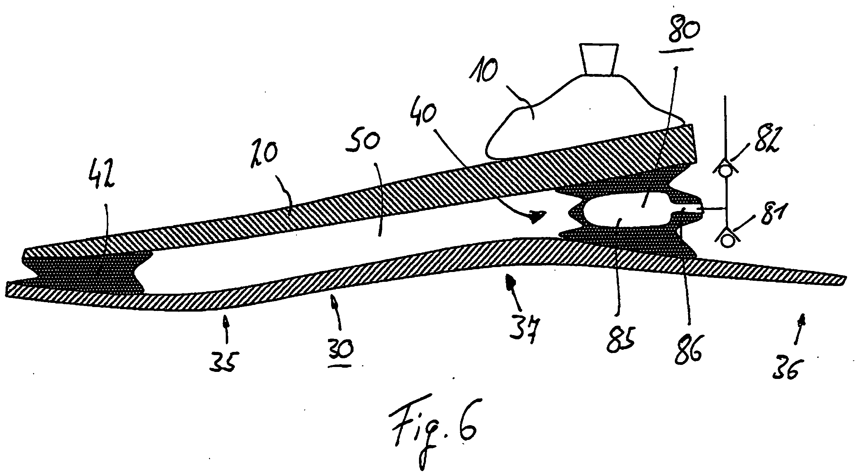

In der Figur 6 ist eine Variante der Erfindung mit einer Vakuumpumpe 80 dargestellt. Der konstruktive Aufbau des Prothesenfußeinsatzes entspricht dem der Figur 3, mit der Veränderung, dass in dem hinteren Koppelelement 40 eine Vakuumpumpe 80 integriert ist. Das hintere Koppelelement 40 ist dabei als Elastomerelement ausgebildet und weist eine elastische Kammer 85 auf, die über einen Verbindungskanal 86 mit der Umgebung in Verbindung steht. Der Verbindungskanal 86 ist in strömungstechnischer Verbindung mit zwei Rückschlagventilen 81, 82 ausgebildet. Das in der Zeichnung dargestellte untere Rückschlagventil 81 sperrt gegen einen Lufteintritt aus der Umgebung, das obere Rückschlagventil 82 sperrt gegen einen Lufteintritt in den zu evakuierenden Bereich der Prothese, beispielsweise einen als Vakuumschaft ausgeführten Prothesenschaft, aus dem Luft aus einem Zwischenraum zwischen dem Schaft und einem Liner abgepumpt wird. Wird das hintere Elastomerelement 40 komprimiert, verringert sich das Volumen der elastischen Kammer 85, so dass die darin befindliche Luft durch den Verbindungskanal 86 und das untere Rückschlagventil 81 in die Umgebung ausgestoßen wird. Wird der Fersenbereich 36 entlastet, entspannt sich das hintere Koppelelement 40, die Dachfeder 20 und die Basisfeder 30 entfernen sich zumindest im Fersenbereich 36 voneinander, so dass sich das Volumen der elastischen Kammer 85 wieder vergrößert. Da keine Luft aus der Umgebung aufgrund der Sperrung durch das untere Rückschlagventil 81 in die Kammer 85 einströmen kann, wird Luft durch das Rückschlagventil 82 und die daran angeschlossene Leitung aus der proximal angeordneten Protheseneinrichtung, insbesondere dem Prothesenschaft abgesaugt.

Statt einer Integration der Vakuumpumpe 80 in das hintere Elastomerelement 40 kann die Vakuumpumpe auch als separate Pumpe ausgebildet und zwischen der Basisfeder 30 und der Dachfeder 20 angeordnet sein. Die Funktionsweise entspricht der oben beschriebenen Funktionsweise, nämlich dass bei einer Verlagerung der beiden Federn 20, 30 aufeinander zu eine Volumenveränderung innerhalb des Kammervolumens stattfindet, die bei einer Volumenverringerung zum Ausstoß in die Umgebung und bei einer Volumenvergrößerung zu einem Ansaugen der Luft aus dem zu evakuierenden Bereich führt. Bei einer Entlastung des Fußes und einer Vergrößerung der Entfernung der beiden Federn 20, 30 voneinander erfolgt dann die umgekehrte Bewegung und Volumenveränderung der Pumpenkammer mit einer entsprechend anderen Pumprichtung. Sofern ein Umlenkmechanismus vorgesehen ist, kann auch bei einer Verlagerung der Federn aufeinander zu eine Volumenvergrößerung und ein Absaugen und bei einer Verlagerung voneinander weg ein Ausstoßen der Luft aus der Kammer 85 erfolgen.

Eine Integration der Vakuumpumpe 80 in den Prothesenfußeinsatz ermöglicht es, die Vakuumpumpe 80 von strukturellen Lasten zu befreien. Der Pumpenhub kann als nützliche Bewegung in die Fußfunktion integriert werden, so dass der bauliche als auch der mechanische Aufwand erheblich verringert und die Bauhöhe des Prothesenfußes erheblich reduziert werden können. Weiterhin kann durch die Integration der Vakuumpumpe 30 in eines der Koppelelemente 40, 42 eine Funktionsübertragung auf die Pumpe erfolgen, beispielsweise können Zugkräfte durch die Vakuumpumpe 80 aufgenommen werden. Darüber hinaus kann durch eine Ansteuerung der Ventile 81, 82 eine Beeinflussung der Fußsteifigkeit und der Einfederrate erfolgen.

## Patentansprüche

1. Prothesenfußeinsatz mit oberen Anschlussmitteln, einer sich von den oberen Anschlussmitteln (10) nach vorn erstreckenden Dachfeder (20) sowie einer Basisfeder (30), die an zumindest zwei Stellen mit der Dachfeder (20) gekoppelt ist, wobei ein hinteres Koppelelement (40) zur Abstützung der Basisfeder (30) vorgesehen und zwischen dem Koppelelement (40), der Dachfeder (20) und der Basisfeder (30) ein Freiraum (50) ausgebildet ist, wobei im Fersenbereich (36) die Basisfeder (30) nach hinten über das hintere Koppelelement (40) als freier Hebel hinausragt, wobei das hintere Koppelelement (40) eine im Wesentlichen senkrecht zur Gehrichtung und parallel zu der Basisfeder (30) verlaufende Schwenkachse für den freien Hebel ausbildet, der bei einem Fersenauftritt um die Schwenkachse verformt wird und die Basisfeder (30) aufgrund der Befestigung an der Dachfeder (20) und dem dadurch zu dem hinteren Koppelelement (40) beabstandeten zweiten Koppelpunkt bei Fersenlast eine 3-Punkt-Biegung ausführt, so dass bei Fersenlast ein Mittelfußbereich (37) der Basisfeder (30) von der Dachfeder (20) weg gebogen wird und der zwischen der Dachfeder (20) und der Basisfeder (30) vorhandene Freiraum (50) sich bei einer Vorderfußbelastung verkleinert, **dadurch gekennzeichnet, dass** die Dachfeder (20) und die Basisfeder (30) über ein als separates Elastomerelement ausgebildetes vorderes Kopplungselement (42) im Zehenbereich aneinander gekoppelt sind.

2. Prothesenfußeinsatz nach Anspruch 1, **dadurch gekennzeichnet, dass** die Basisfeder (30) eine im Vorderfußbereich (35) zum Boden konvexe Kontur aufweist.

3. Prothesenfußeinsatz nach Anspruch 1 oder 2, **dadurch gekennzeichnet, dass** die Basisfeder (30) und die Dachfeder (20) als Doppelfederbogen ausgebildet sind und sich die Dachfeder (20) als oberer Bogen bis zum Fersenbereich (36) der Basisfeder (30) erstreckt.

4. Prothesenfußeinsatz nach einem der voranstehenden Ansprüche, **dadurch gekennzeichnet, dass** sich an den nach unten gewölbten Vorderfußbereich (35) der Basisfeder (30) ein nach oben gewölbter Mittelfußbereich (37) anschließt.

5. Prothesenfußeinsatz nach einem der voranstehenden Ansprüche, **dadurch gekennzeichnet, dass** das hintere Koppelelement (40) als ein Elastomerelement ausgebildet ist.

6. Prothesenfußeinsatz nach einem der voranstehenden Ansprüche, **dadurch gekennzeichnet, dass** das vordere Koppelelement (42) zwischen dem vorderen Endbereich der Basisfeder (30) und dem nach oben ansteigenden Teil der Basisfeder (30) angeordnet ist.

7. Prothesenfußeinsatz nach einem der voranstehenden Ansprüche, **dadurch gekennzeichnet, dass** zwischen der Basisfeder (30) und der Dachfeder (20) eine Vakuumpumpe (80) angeordnet ist.

8. Prothesenfußeinsatz nach Anspruch 7, **dadurch gekennzeichnet, dass** die Vakuumpumpe (80) als Koppelelement (40, 42) oder Teil eines Koppelelementes (40, 42) ausgebildet ist.

9. Prothesenfuß mit einem Prothesenfußeinsatz nach einem der voranstehenden Ansprüche und einer Prothesenfußkosmetik (70) mit einem Sohlenbereich (75), der unterschiedliche Materialstärken aufweist.

10. Prothesenfuß nach Anspruch 9, **dadurch gekennzeichnet, dass** der Sohlenbereich (75) der Prothesenfußkosmetik (70) im Vorderfußbereich (35) und/oder Fersenbereich (36) dicker als im Mittelfußbereich (37) ausgebildet ist.

## Claims

1. A prosthetic foot insert with upper attachment means, a roof spring (20) extending forward from the upper attachment means (10), and a base spring (30) coupled to the roof spring (20) at at least two points, wherein a rear coupling element (40) is provided for supporting the base spring (30), and a free space (50) is formed between the coupling element (40), the roof spring (20) and the base spring (30) and wherein the base spring (30), in the heel area (36), protrudes rearward as a free lever beyond the rear coupling element (40), wherein the rear coupling element (40) forms a pivot axis for the free lever, which is deformed about the pivot axis at heel strike, which pivot axis extends substantially perpendicularly with respect to the direction of walking and parallel to the base spring (30), wherein the base spring (30), due to the securing to the roof spring (20) and the second coupling point being thereby at a distance from the rear coupling element (40), performs a three-point-bending when the heel is loaded, so that a middle foot portion (37) of the base spring (30) is bent away from the roof spring (20) and the free space (50) present between the roof spring (20) and the base spring (30) decreases in size when the forefoot is loaded, **characterized in that** the roof spring (20) and the base spring (30) are coupled to each other in the toe area via a front coupling element (42), which is designed as separate elastomeric element.

2. The prosthetic foot insert as claimed in claim 1, **characterized in that** the base spring (30) has a contour that is convex to the ground in the forefoot area (35).

3. The prosthetic foot insert as claimed in claim 1 or 2, **characterized in that** the base spring (30) and the roof spring (20) are designed as a double spring arch, and the roof spring (20) extends as the upper arch as far as the heel area (36) of the base spring (30).

4. The prosthetic foot insert as claimed in one of the preceding claims, **characterized in that** the downwardly bulging forefoot area (35) of the base spring (30) is adjoined by an upwardly bulging mid-foot area (37).

5. The prosthetic foot insert as claimed in one of the preceding claims, **characterized in that** the rear coupling element (40) is designed as a an elastomeric element.

6. The prosthetic foot insert as claimed in one of the preceding claims, **characterized in that** a front coupling element (42) is arranged between the front end area of the base spring (30) and the upwardly sloping part of the base spring (30).

7. The prosthetic foot insert as claimed in one of the preceding claims, **characterized in that** a vacuum pump (80) is arranged between the base spring (30) and the roof spring (20).

8. The prosthetic foot insert as claimed in claim 7, **characterized in that** the vacuum pump (80) is designed as a coupling element (40, 42) or is part of a coupling element (40, 42).

9. The prosthetic foot with a prosthetic foot insert as claimed in one of the preceding claims and with a prosthetic foot cover (70) having a sole area (75) with different material thicknesses.

10. The prosthetic foot as claimed in claim 9, **characterized in that** the sole area (75) of the prosthetic foot cover (70) is thicker in the forefoot area (35) and/or heel area (36) than it is in the mid-foot area (37).

## Revendications

1. Insert de pied de prothèse comprenant des organes de raccordement supérieurs, un ressort de coiffe (20) s'étendant vers l'avant depuis les organes de raccordement supérieurs (10) ainsi qu'un ressort de base (30) qui est couplé avec le ressort de coiffe (20) en au moins deux emplacements, dans lequel il est prévu un élément de couplage postérieur (40) pour soutenir le ressort de base (30) et un espace libre (50) est réalisé entre l'élément de couplage (40), le ressort de coiffe (20) et le ressort de base (30), et dans lequel, dans la zone de talon (36), le ressort de base (30) dépasse vers l'arrière au-delà de l'élément de couplage postérieur (40) en formant un levier libre, et l'élément de couplage postérieur (40) forme un axe de pivotement, s'étendant sensiblement perpendiculairement à la direction de marche et parallèlement au ressort de base (30), pour le levier libre, lequel se déforme autour de l'axe de pivotement lors d'une attaque du talon, et dans lequel le ressort de base (30) exécute, en raison de la fixation sur le ressort de coiffe (20) et en raison du second point de couplage écarté pour cette raison par rapport à l'élément de couplage postérieur (40), une flexion en trois points lors de l'application d'une charge au talon, de sorte que lors d'une application d'une charge au talon, une zone de pied médiane (37) du ressort de base (30) est fléchie en éloignement du ressort de coiffe (20), et l'espace libre (50) présent entre le ressort de coiffe (20) et le ressort de base (30) diminue lors de l'application d'une charge sur l'avant-pied, **caractérisé en ce que** le ressort de coiffe (20) et le ressort de base (30) sont couplés l'un à l'autre au niveau des orteils par un élément de couplage antérieur (40, 42) réalisé en tant qu'élément en élastomère séparé.

2. Insert de pied de prothèse selon la revendication 1, **caractérisé en ce que** le ressort de base (30) présente un contour convexe vers le sol dans la zone de l'avant-pied (35).

3. Insert de pied de prothèse selon la revendication 1 ou 2, **caractérisé en ce que** le ressort de base (30) et le ressort de coiffe (20) sont réalisés sous forme d'un arceau-ressort double et le ressort de coiffe (20) s'étend, sous forme d'un arceau supérieur, jusqu'à la zone de talon (36) du ressort de base (30).

4. Insert de pied de prothèse selon l'une des revendications précédentes, **caractérisé en ce qu'**une zone de pied médiane (37) bombée vers le haut se raccorde à la zone d'avant-pied (35), bombée vers le bas, du ressort de base (30).

5. Insert de pied de prothèse selon l'une des revendications précédentes, **caractérisé en ce que** l'élément de couplage postérieur (40) est réalisé comme un élément en élastomère.

6. Insert de pied de prothèse selon l'une des revendications précédentes, **caractérisé en ce que** l'élément de couplage antérieur (42) est agencé entre la zone terminale antérieure du ressort de base (30) et la partie de ressort de base (30) qui monte vers le haut.

7. Insert de pied de prothèse selon l'une des revendications précédentes, **caractérisé en ce qu'**une pompe à vide (80) est agencée entre le ressort de base (30) et le ressort de coiffe (20).

8. Insert de pied de prothèse selon la revendication 7, **caractérisé en ce que** la pompe à vide (80) est réalisée sous forme d'élément de couplage (40, 42) ou d'une partie d'un élément de couplage (40, 42).

9. Pied de prothèse comprenant un insert de pied de prothèse selon l'une des revendications précédentes, et un élément esthétique de pied de prothèse (70) avec une zone de semelle (75) qui présente des matériaux d'épaisseurs différentes.

10. Pied de prothèse selon la revendication 9, **caractérisé en ce que** la zone de semelle (75) de l'élément esthétique de pied de prothèse (70) est réalisée plus épaisse dans la zone d'avant-pied (35) et/ou dans la zone de talon (36) que dans la zone médiane du pied (37).
